Europäisches Patentamt

European Patent Office

Office européen des brevets

(19)

(11) Veröffentlichungsnummer: **0 002 064**
**B1**

(12) **EUROPÄISCHE PATENTSCHRIFT**

(45) Veröffentlichungstag der Patentschrift:
21.09.83

(51) Int. Cl.³: **A 61 F 1/00,** A 61 K 6/00

(21) Anmeldenummer: **78101410.5**

(22) Anmeldetag: **20.11.78**

(54) **Gemisch mit schäumendem und resorbierbarem Effekt zur Herstellung eines Spongiosazementes und Spongiosazement als Implantat bei chirurgischen Eingriffen.**

(30) Priorität: **23.11.77 DE 2752297**

(43) Veröffentlichungstag der Anmeldung:
**30.05.79 Patentblatt 79/11**

(45) Bekanntmachung des Hinweises auf die Patenterteilung:
**21.09.83 Patentblatt 83/38**

(84) Benannte Vertragsstaaten:
**CH FR GB**

(56) Entgegenhaltungen:
**CH-A- 165 738**
**DE-A-1 933 917**
**FR-A-2 361 211**

(73) Patentinhaber: **Tomic, Dobrivoje, Dr.,**
**Fasangartenstrasse 159, D-8000 München 90 (DE)**

(72) Erfinder: **Tomic, Dobrivoje, Dr., Fasangartenstrasse 159,**
**D-8000 München 90 (DE)**

ACTORUM AG

**Gemisch mit schäumendem und resorbierbarem Effekt zur Herstellung eines Spongiosazementes und Spongiosazement als Implantat bei chirurgischen Eingriffen**

Die Erfindung betrifft ein Gemisch mit schäumendem und resorbierbarem Effekt zur Herstellung eines Spongiosazementes für die Verbindung, Verwachsung oder den Ersatz von menschlichem oder tierischen Hartgewebe, Knochenteilen oder Zahnwurzeln sowie einen solchen Spongiosazement als Implantat bei chirurgischen Eingriffen.

Als gewebsfreundliches Material zum Knochenersatz und zur Befestigung von Fremdkörpern im Knochen sind einige sog. Knochenzemente auf Polymethylmethacrylat-Basis seit langem bekannt («Die Knochenzemente», Enke Verlag Stuttgart, 1975). Ihr Verbleiben im Knochen wird durch mechanisch geschaffene Retentionen möglich und es besteht stellenweise nur ein Berührungskontakt zwischen dem Knochenzement und Knochengewebe. Nach dem Erhärten entsteht eine geschlossene glatte Fläche, die unverändert bleibt.

Alle bekannten Knochenzemente sind jedoch mit zahlreichen Nachteilen verbunden, insbesondere bei der Verankerung und Verwachsung mit umliegendem Gewebe. Bei ihrer Fixierung wird sehr viel gesunde Knochensubstanz geopfert und trotzdem wird nur stellenweise Kontakt und Anlehnung an umliegende Knochenteile erreicht. Bei bekannten Knochenzementen ist ein Verwachsen der umliegenden Gewebe im Knochenzement nicht möglich, was aber für Stabilität und Verbleiben im Knochen sehr nachteilig ist.

Aufgabe der Erfindung ist es, ein Gemisch zur Herstellung eines Spongiosazementes mit schäumenden, haftenden und resorbierbaren Eigenschaften zu schaffen, der sich beim Applizieren rasch und vollkommen mit dem Knochengewebe verbindet und dabei spongiosaartige Stellen zur Ossifikation und zum Eindringen von hartem und weichem Gewebe aufweist und selbst resorbierbare Substanzteile besitzt, so dass sowohl die gewünschte Festigkeit des Zementes als auch seine optimale Inkorporierung im Organismus erhalten wird.

Die Lösung dieser Aufgabe ist ein Gemisch aus einer pulverförmigen Ausgangsmischung als Trägerstoff, insbesondere auf der Basis von Polymethylmethacrylat unter Zusatz eines flüssigen Monomers, mit weiteren Mischungsbestandteilen, welche die flüssige Mischung aufschäumen und im festgewordenen Zustand den geschäumten Zement ergeben, wobei dem Gemisch gegebenenfalls noch ein Bindemittel hinzugefügt ist.

Auf Grund der einfachen Zusammensetzung des erfindungsgemässen schäumenden Spongiosazementes, und auf Grund des leichten und kontrollierbaren Schäumungseffektes und der Resorbierbarkeit von beigemischten Substanzen ist die Applizierung des erfindungsgemässen Zementes im plastischen und im festen Zustand möglich. Der Schäumungsprozess wird mit Einwirkung von Säure auf den Zement in Gang gesetzt und sein Umfang ist von Konzentration bzw. Prozentsatz beteiligter Substanzen abhängig. Hierfür als Träger und Gerüst verwendetes Polymethylmetacrylat (PMMA) bleibt unverändert.

Bei Einwirken von Phosphorsäure auf den erfindungsgemässen Spongiosazement kommt es sofort zur Schäumung von $CaCO_3$ und Befreiung von $CO_2$, wodurch gleichzeitig spongiosaartige Flächen entstehen. Wird dieser Vorgang im Knochen vorgenommen, so kommt es gleichzeitig auch zur Ätzung und Anrauhung bzw. Bildung von adhäsiven Flächen, was zu einer Verzahnung und totalen Verbindung untereinander führt.

Dieser Inkorporierungsvorgang wird dann in etwa 8 Wochen durch das Eindringen und Verwachsen vom Gewebe in die Lagunen und porösen Stellen beendet, wobei durch die Beimischung von einer resorbierbaren Substanz wie $Na_2HPO_4$ oder anderen Stoffen noch zusätzliche freie Löcher für das Eindringen von Gewebe entstehen. Der so eingesetzte erfindungsgemässe schäumend-resorbierbare Spongiosazement erreicht im Organismus bzw. Knochengewebe seine optimale Integrierung und Inkorporierung. Die Bestandteile des erfindungsgemässen Zementes können in variablen Mengenanteilen vorliegen

Eine Zusammensetzung des Pulvers zur Herstellung eines Spongiosazementes, wie am Schluss angegeben, ist besonders vorteilhaft, insbesondere wenn dieser im Kiefer und als Zahnwurzelersatz eingesetzt wird. Die Versuche und Erfolge in der Kieferchirurgie geben Anlass zur möglichen Anwendung des schäumend-resorbierbaren Spongiosazementes auch in der Orthopädie, Unfallchirurgie und auf anderen Gebieten.

Zur Herstellung des erfindungsgemässen Spongiosazementes erfolgt das Einverleiben und Vermischen der einzelnen Komponenten in bekannter, auf pharmazeutischem Gebiet üblicher Weise. Zur Herstellung des Pulvers wird zweckmässig vom angegebenen bekannten PMMA-Pulver ausgegangen. Diesem werden die erforderlichen Komponenten, $CaCO_3$ und $Na_2HPO_4$, ggf. in vorgemischter Form einverleibt. Dem Pulver wird ein Monomer im flüssigen Zustand zugesetzt. Der erfindungsgemässe Zement in angegebener Form eignet sich als selbständiges Implantat, Ersatz für hartes Gewebe, als Befestigungs- und Verbindungsmittel von hartem Gewebe untereinander und/oder zu anderen Fremdkörpern sowie als Beschichtungssubstanz. Bei Anwendung der erfindungsgemässen Spongiosazemente im Knochengewebe kommt neben den bereits aufgeführten Vorteilen der weitere Vorteil hinzu, dass z.B. ein Implantat seine Kontaktfläche zum Knochen wesentlich erweitert, was für Funktion und Belastung derselben von grosser Bedeutung ist.

Das folgende Beispiel soll die Erfindung näher erläutern.

Beispiel

Dieses Beispiel zeigt eine bevorzugte Zusammensetzung der erfindungsgemässen Pulver-Mischung zum Vermischen mit einem flüssigen Monomer, wie oben angegeben.

| Komponente | ungefährer Gehalt | ungefährer Gehalt in % |
|---|---|---|
| PMMA | 7,0 g | 70 |
| CaCO$_3$ | 2,0 g | 20 |
| Na$_2$HPO$_4$·12H$_2$O | 1,0 g | 10 |

**Patentansprüche**

1. Gemisch zur Herstellung eines Spongiosazements für die Verbindung, Verwachsung oder den Ersatz von menschlichem oder tierischem Hartgewebe, Knochenteilen oder Zahnwurzeln bei chirurgischen Eingriffen, bestehend aus einer pulverförmigen Ausgangsmischung als Trägerstoff, insbesondere auf der Basis von Polymethylmethacrylat, unter Zusatz eines flüssigen Monomers, gekennzeichnet durch weitere Mischungsbestandteile, welche die flüssige Mischung aufschäumen und im fest gewordenen Zustand den geschäumten Zement ergeben, wobei dem Gemisch gegebenenfalls noch ein Bindemittel beigefügt ist.

2. Gemisch nach Anspruch 1, gekennzeichnet durch einen weiteren Zusatz in Form von resorbierbaren Substanzen.

3. Gemisch nach Anspruch 1 und 2, dadurch gekennzeichnet, dass neben Polymethylmethacrylat als Trägerstoff und Na$_2$HPO$_4$ als resorbierbare Substanz noch CaCO$_3$ als schäumende Substanz unter Beigaben von Phosphorsäure vorgesehen ist.

4. Gemisch nach Anspruch 3, dadurch gekennzeichnet, dass das CaCO$_3$ mit einem Anteil von 10–50% enthalten ist.

5. Gemisch nach Anspruch 4, gekennzeichnet durch ein Mischungsverhältnis von etwa 20% CaCO$_3$, etwa 70% Polymethylmethacrylat und etwa 10% Na$_2$HPO$_4$.

6. Spongiosazement nach Ansprüchen 1 bis 5 zur Verwendung als Implantat zum Ersatz und zur Verbindung von Knochenteilen oder Zahnwurzeln bzw. zur Befestigung und Inkorporierung von Fremdkörpern bei chirurgischen Eingriffen.

**Claims**

1. Mixture for the manufacture of a spongiosa cement for the connection, coalescence, or replacement of human or animal hard tissue, sections of bone or roots of teeth in surgical procedures, composed of a powdery mixture as supporting substance, mainly on the base of Polymethylmethacrylat with addition of a liquid monomer, indentifiable by the fact that it contains other agents which have an effervescent effect on the liquid substance and produces the effervescent cement in a solid state, in the case of which a bonding material may be used.

2. Material in accordance with Claim 1, idetifiable by the presence of additional reapsorptive substances.

3. Material in accordance with Claims 1 and 2 identifiable by the fact that, besides Polymethylmethacrylat as medium an Na$_2$HPO$_4$ as reapsorptive substance, CaCO$_3$ with the addition of a phosphoric acid has been provided for.

4. Material in accordance with Claim 3, identifiable by a content of 10%–50% of CaCO$_3$.

5. Material in accordance with Claim 4, identifiable by a proportion of approximately 20% CaCO$_3$, approximately 70% Polymethylmethacrylat, and approximately 10% Na$_2$HPO$_4$.

6. Application of the spongiosa cement in accordance with Claims 1 through 5 as implant for the replacement for and cementing of sections of bones or roots of teeth, or in the fastening and incorporation of foreign bodies in surgical procedures.

**Revendications**

1. Mélange servant à préparer un ciment à substance spongieuse utilisé lors d'interventions chirurgicales pour la liaison, la soudure ou le remplacement de tissus osseux compacts humains ou animaux, de fragments d'os ou de racines de dents, composé d'un mélange initial en poudre comme matière de base, surtout à partir de polyméthylméthacrylate, additionné d'un monomère liquide et caractérisé par d'autres ingrédients de mélange, lesquels font mousser le mélange liquide et lesquels, à l'état solide, donnent le ciment moussé. En outre, un liant peut, le cas échéant, être ajouté au mélange.

2. Mélange d'après demande 1, caractérisé par une addition supplémentaire sous forme de substrances résorbables.

3. Mélange d'après demandes 1 et 2, caractérisé par le fait que, en plus du polyméthylméthacrylate comme matière de base et en plus du Na$_2$HPO$_4$ comme substance résorbable, du CaCO$_3$ est prévu comme substance effervescente, avec addition d'acide phosphorique.

4. Mélange d'après demande 3, caractérisé par le fait que la part du CaCO$_3$ est de 10 à 50%.

5. Mélange d'après demande 4, caractérisé par un dosage avec environ 20% de CaCO$_3$, environ 70% de polyméthylméthacrylate et environ 10% de Na$_2$HPO$_4$.

6. Ciment à substance spongieuse d'après demandes 1 à 5, à utiliser comme implant pour le remplacement et la liaison de fragments d'os ou de racines de dents ou pour la fixation et l'incorporation de corps étrangers lors d'interventions chirurgicales.